# EUROPEAN PATENT SPECIFICATION

(11) **EP 4 023 772 B1**
(45) Date of publication and mention of the grant of the patent: **28.02.2024**
(21) Application number: 20932880.6
(22) Date of filing: 14.10.2020
(51) Int. Cl.: C12Q 1/70, C12Q 1/6851

(54) **COMPOSITION FOR DETECTING MUTATIONS OF 2019 NOVEL CORONAVIRUS, USE AND KIT THEREOF**
ZUSAMMENSETZUNG ZUM DETEKTIEREN VON MUTATIONEN DES NEUEN CORONAVIRUS 2019, VERWENDUNG UND KIT DAFÜR
COMPOSITION POUR LA DÉTECTION DE MUTATIONS DU NOUVEAU CORONAVIRUS 2019, UTILISATION ET KIT ASSOCIÉS

(30) Priority: 29.04.2020 CN 202010358731
(43) Date of publication of application: 06.07.2022
(73) Proprietor: Sansure Biotech Inc., Changsha, Hunan 410205 (CN)
(72) Inventor: DAI, Lizhong, Changsha, Hunan 410205 (CN); DENG, Zhongping, Changsha, Hunan 410205 (CN); YAN, Jin, Changsha, Hunan 410205 (CN); CHEN, Shiyao, Changsha, Hunan 410205 (CN); TAN, Deyong, Changsha, Hunan 410205 (CN); LIU, Jia, Changsha, Hunan 410205 (CN); REN, Xiaomei, Changsha, Hunan 410205 (CN); GUO, Xinwu, Changsha, Hunan 410205 (CN)
(74) Representative: Grünecker Patent- und Rechtsanwälte PartG mbB
(86) International application number: PCT/CN2020/120898
(87) International publication number: WO 2021/218042

(56) References cited:
- CN-A- 110 982 943
- CN-A- 111 074 005
- CN-A- 111 304 372
- US-A1- 2020 102 606
- Deng Xianding ET AL: "A Genomic Survey of SARS-CoV-2 Reveals Multiple Introductions into Northern California without a Predominant Lineage", medRxiv, 30 March 2020 (2020-03-30), pages 1-16, XP055883883, DOI: 10.1101/2020.03.27.20044925 Retrieved from the Internet: URL:https://www.ncbi.nlm.nih.gov/pmc/artic les/PMC7276006/pdf/nihpp-2020.03.27.200449 25.pdf [retrieved on 2022-01-26] & Deng Xianding: "A Genomic Survey of SARS-CoV-2 Reveals Multiple Introductions into Northern California without a Predominant Lineage - primer sequences S1", , 30 March 2020 (2020-03-30), XP093008327, Retrieved from the Internet: URL:https://www.medrxiv.org/content/10.110 1/2020.03.27.20044925v1.supplementary-mate rial [retrieved on 2022-12-14]
- WANG CHANGTAI: "The establishment of reference sequence for SARS-CoV-2 and variation analysis", JOURNAL OF MEDICAL VIROLOGY, vol. 92, no. 6, 13 March 2020 (2020-03-13) , pages 667-674, XP055860448,
- LI JIANGUO ET AL: "Bayesian phylodynamic inference on the temporal evolution and global transmission of SARS-CoV-2", JOURNAL OF INFECTION, ACADEMIC PRESS, LONDON, GB, vol. 81, no. 2, 20 April 2020 (2020-04-20) , pages 318-356, XP086228433, ISSN: 0163-4453, DOI: 10.1016/J.JINF.2020.04.016 [retrieved on 2020-04-20]
- Farkas Carlos ET AL: "Insights on early mutational events in SARS-CoV-2 virus reveal founder effects across geographical regions", bioRxiv, 12 April 2020 (2020-04-12), XP093008274, DOI: 10.1101/2020.04.09.034462 Retrieved from the Internet: URL:https://www.biorxiv.org/content/biorxi v/early/2020/04/12/2020.04.09.034462.full. pdf [retrieved on 2022-12-14]
- SAH RANJIT ET AL: "Complete Genome Sequence of a 2019 Novel Coronavirus (SARS-CoV-2) Strain Isolated in Nepal", GENOME ANNOUNCEMENTS, vol. 9, no. 11, 12 March 2020 (2020-03-12) , pages e00169-20, XP055862498, US ISSN: 2169-8287, DOI: 10.1128/MRA.00169-20
- JIANNIS RAGOUSSIS: "Genotyping Technologies for Genetic Research", ANNUAL REVIEW OF GENOMICS AND HUMAN GENETICS, vol. 10, no. 1, 1 September 2009 (2009-09-01), pages 117-133, XP055116403, ISSN: 1527-8204, DOI: 10.1146/annurev-genom-082908-150116
- M. H. HAZBON ET AL: "Hairpin Primers for Simplified Single-Nucleotide Polymorphism Analysis of Mycobacterium tuberculosis and Other Organisms", JOURNAL OF CLINICAL MICROBIOLOGY, vol. 42, no. 3, 1 March 2004 (2004-03-01), pages 1236-1242, XP055432518, US ISSN: 0095-1137, DOI: 10.1128/JCM.42.3.1236-1242.2004
- Victor M Corman, Olfert Landt, Marco Kaiser, Richard Molenkamp, Adam Meijer, Daniel Kw Chu, Tobias Bleicker, Sebastian Brünin: "Detection of 2019 novel coronavirus (2019-nCoV) by real-time RT-PCR", Eurosurveillance, Centre Europeen pour la Surveillance Epidemiologique du SIDA, FR, vol. 25, no. 3, 23 January 2020 (2020-01-23), XP055695049, FR ISSN: 1560-7917, DOI: 10.2807/1560-7917.ES.2020.25.3.2000045

## Description

### Technical Field

The present invention relates to the field of molecular biological detection, and in particular, to the detection field of 2019 novel coronavirus, and more particularly, it can detect two base mutations of 2019 novel coronavirus (8782C→T and 28144T→C).

### Background

2019 novel coronavirus (SARS-COV-2) is the pathogen that causes novel coronavirus pneumonia (COVID-19). The virus has begun to circulate in the second half of 2019 and spread rapidly, with the World Health Organization declaring a global pandemic on March 11, 2020. Up to April 01, 2020, the cumulative number of confirmed COVID-19 cases worldwide has exceeded 800,000, and the trend is worsening.

Common signs of the COVID-19 include respiratory symptoms such as fever, cough, shortness of breath, and dyspnea. In more severe cases, the infection can lead to pneumonia, severe acute respiratory syndrome, kidney failure, and further death. There is currently no specific treatment for 2019 novel coronavirus-induced diseases, but many symptoms can be treated with complementary care and supportive treatment.

Xiaolu Tang et al. have found that there is a linkage between the bases at the 8782-nd and 28144-th sites through the full gene analysis of the virus (101/103, 98%). For example, according to the types of the two bases, the novel coronavirus has two haplotypes: L type (C8782 and T28144) and S type (T8782 and C28144). The L type and S type are named from different amino acids encoded through changes of the 28144-site base; codon where the L-type T28144 is encodes Leucine, and codon where the S-type C28144 is encodes Serine.

Deng Xianding ET AL, "A Genomic Survey of SARS-CoV-2 Reveals Multiple Introductions into Northern California without a Predominant Lineage", medrxiv, pages 1 - 16, and Deng Xianding, "A Genomic Survey of SARS-CoV-2 Reveals Multiple Introductions into Northern California without a Predominant Lineage - primer sequences S1", supplementary-material relates to phylogenetic analyses to reveal different SARS-CoV-2-lineages. SARS-CoV-2 sequences analyzed therein all shared the two single nucleotide polymorphisms C17747T and A17858G, as well as the "triad" of single nucleotide polymorphisms common to all Washington state (WA1) lineages, i.e. C8782T, T28144C and C18060T.

WANG CHANGTAI, "The establishment of reference sequence for SARS-CoV-2 and variation analysis", JOURNAL OF MEDICAL VIROLOGY, vol. 92, no. 6, pages 667 - 674 relates to phylogenetic and homology analyses along the SARS-CoV-2 genome.

LI JIANGUO ET AL, "Bayesian phylodynamic inference on the temporal evolution and global transmission of SARS-CoV-2", JOURNAL OF INFECTION, ACADEMIC PRESS, LONDON, GB, vol. 81, no. 2, pages 318 - 356 relates to the safety and clinical impact of Baricitinib therapy in COVID-19.

Farkas Carlos ET AL, "Insights on early mutational events in SARS-CoV-2 virus reveal founder effects across geographical regions", bioRxiv describes early mutational events across samples from publicly available SARS-CoV-2 sequences.

SAH RANJIT ET AL, "Complete Genome Sequence of a 2019 Novel Coronavirus (SARS-CoV-2) Strain Isolated in Nepal", GENOME ANNOUNCEMENTS, US, (20200312), vol. 9, no. 11, pages e00169 - 20 relates to the genome sequence of a SARS-CoV-2 strain isolated in Nepal.

JIANNIS RAGOUSSIS, "Genotyping Technologies for Genetic Research", ANNUAL REVIEW OF GENOMICS AND HUMAN GENETICS, vol. 10, no. 1, pages 117 - 133 relates to various technologies for genetic research.

M. H. HAZBON ET AL, "Hairpin Primers for Simplified Single-Nucleotide Polymorphism Analysis of Mycobacterium tuberculosis and Other Organisms", JOURNAL OF CLINICAL MICROBIOLOGY, US, vol. 42, no. 3, pages 1236 - 1242 relates to a method for the detection of single-nucleotide polymorphisms (SNPs) in *Mycobacterium tuberculosis.*

US 2020/102606 A1 relates to digital polymerase chain reaction (PCR) primer-pair constructs having discrimination power in detecting nucleic acid sequence variations among different nucleic acids.

Upon comparative analysis of data, the L type and S type are different in the rate of severe disease and infectious ability. This provides the basis for refining the diagnosis of the COVID-19, which means that through genotyping detection, the type of virus in a patient can clinically be determined, and clinical features and disease progression can be predicted, to develop a more targeted treatment solution accordingly.

Hence, detecting and recognizing the bases at two sites of the 2019 novel coronavirus have positive significance for epidemic prevention and control and the treatment of confirmed patients. However, currently no corresponding detection reagent exists yet.

In this field, a related product capable of detecting and recognizing bases at two sites 8782 and 28144 of the 2019 novel coronavirus.

### Summary

In view of the above, the present invention provides a composition for detecting mutations of 2019 novel coronavirus, including:
a first nucleic acid composition:
   8782 C-type forward primer: 5'-GCTGATTTTGACACATGGTTCATC-3' (SEQ ID NO: 1); and
   28144 T-type forward primer: 5'-ATCGGTAATTATACAGTTTCCAGATT-3' (SEQ ID NO: 2); and
a second nucleic acid composition:
   8782 T-type forward primer: 5'-GCTGATTTTGACACATGGTATCGT-3' (SEQ ID NO: 3); and
   28144 C-type forward primer: 5'-ATCGGTAATTATACAGTTTCCTTTGC-3' (SEQ ID NO: 4);
where each nucleic acid composition further includes:
   8782 reverse primer: 5'-CCATTAGTTGTGCGTAATATCGT-3' (SEQ ID NO: 5);
   8782 probe: 5'-CTTGCCCATTGATTGCTGCAGTCATAA-3' (SEQ ID NO: 6);
   28144 reverse primer: 5'-CAACACGAACGTCATGATACTCTA-3' (SEQ ID NO: 7); and
   28144 probe: 5'-ATTGCCAGGAACCTAAATTGGGTAGT-3' (SEQ ID NO: 8).

Using the composition may distinguish that the 8782 site of the 2019 novel coronavirus is C type or T type, and may distinguish that 28144 is T or C type. By detecting the types of bases at two sites, S type and L type of the novel coronavirus can be distinguished, for example, to provide the basis for refining the diagnosis of coronavirus disease 2019 (COVID-19), determine the virus type of a patient, and predict clinical features and disease progression to update and improve treatment plan accordingly, thereby having positive significance for epidemic prevention and control and the treatment of confirmed patients.

By using the composition of the present invention, the Ct value of a primer detection amplification specific target is small, and the amplification efficiency thereof is high; a difference between the Ct value of a specific template for primer detection and the Ct value of a non-specific template is relatively large, and the specificity thereof is good. Moreover, using the comparison example of the composition of the present invention, the Ct value of the primer detection amplification specific coarse target is greater than that of the composition of the present invention, and the amplification efficiency thereof is relatively low; at the same time, a difference between the Ct value of the specific template and the Ct value of the non-specific template is smaller than that of the composition of the present invention, and the specificity thereof is relatively poor.

The composition of the present invention can be applied to fluorescence quantitative PCR detection.

Furthermore, in the composition, the 8782 probe and the 28144 probe carry fluorescent reporter groups that do not interfere with each other.

In the text, "not interfere with each other" means that the fluorescent reporter groups used by the probes in the composition are different, and may not affect each other's detection, that is, different channels can be used for detection. For example, FAM, HEX, ROX and CY5 can be used. These groups do not have close absorbance values and can select different channels, and thus may not interfere with each other.

Furthermore, the composition includes an internal standard upstream primer, an internal standard downstream primer and an internal standard probe for monitoring.

In the present invention, the fluorescent reporter group can be selected from a group consisting ofFAM, HEX, ROX, VIC, CY5, 5-TAMRA, TET, CY3, and JOE, and is not limited thereto.

In a specific implementation, the fluorescent reporter group of the first 8782 as shown in SEQ ID NO: 6 is HEX; and the fluorescent reporter group of the 28144 probe as shown in SEQ ID NO: 8 is FAM.

Furthermore, the 3' end of the probe also has a quenching group such as BHQ1 or BHQ2.

Furthermore, the dosage of the primer in the composition is 25-800 nM, and the dosage of the probe in the composition is 10-500 nM.

In a specific implementation, the two nucleic acid compositions combined in the present invention are respectively present in a separate package.

Furthermore, each ingredient of the composition of the present invention presents in a mixed manner.

In a second aspect, the present invention provides use of the composition of the present invention in preparation of a kit for detecting mutations of 2019 novel coronavirus.

In a third aspect, the present invention provides a kit for detecting mutations of 2019 novel coronavirus, including the composition of the present invention.

Furthermore, the kit further includes at least one of dNTP, PCR buffer, and Mg²⁺.

The common PCR buffer is composed of buffer systems such as Tris-HCl, MgCh, KC1, and TritonX-100. In general, the total volume of a single PCR reaction tube is 20-200 µl.

In a specific implementation, the specific ingredients of the first nucleic acid composition of the present invention are as shown below:

| Material | Concentration | Single reaction dosage (µL) |
|---|---|---|
| RT-PCR buffer | 5X | 10 |
| H₂O | 100% | 7.6 |
| MGCl₂ | 50 mM | 3 |
| DNTP | 25 mM (each) | 3 |
| 8782 C-type forward primer | 50 µM | 0.5 |
| 8782 C-type reverse primer | 50 µM | 0.5 |
| 8782 probe | 50 µM | 0.2 |
| 28144 T-type forward primer | 50 µM | 0.5 |
| 28144 C-type reverse primer | 50 µM | 0.5 |
| 28144 probe | 50 µM | 0.2 |
| Total volume | | 26 |

In a specific implementation, the specific ingredients of the second nucleic acid composition of the present invention are as shown below:

| Material | Concentration | Single reaction dosage (µL) |
|---|---|---|
| RT-PCR buffer | 5X | 10 |
| H₂O | 100% | 7.6 |
| MgCl₂ | 50 mM | 3 |
| dNTP | 25 mM (each) | 3 |
| 8782 T-type forward primer | 50 µM | 0.5 |
| 8782 C-type reverse primer | 50 µM | 0.5 |
| 8782 probe | 50 µM | 0.2 |
| 28144 C-type forward primer | 50 µM | 0.5 |
| 28144 C-type reverse primer | 50 µM | 0.5 |
| 28144 probe | 50 µM | 0.2 |
| Total volume | | 26 |

Furthermore, the kit further includes at least one of a nucleic acid release reagent, reverse transcriptase, and DNA polymerase.

Furthermore, the detection primer in the composition is 25-800 nM, and the dosage of the probe in the composition is 10-500 nM.

Furthermore, the concentration of the reverse transcriptase is 5-15 U/µL. For example, the reverse transcriptase can be murine leukemia reverse transcriptase (MMLV) or Tth enzyme. The concentration of the DNA polymerase is 3-15 U /µL. For example, the DNA polymerase can be Taq enzyme.

Furthermore, the kit further includes a positive control. The positive control includes sequences near an 8782-nd site and sequences near a 28144-th site of a 2019 novel coronavirus genome, and the 8782-nd site is C or T, and the 28144-th site is C or T. For example, the 8782-nd site is C and the 28144-th site is T. The 8782-nd site is T and the 28144-th site is C. The 8782-nd site is C and the 28144-th site is C. The 8782-nd site is T and the 28144-th site is T.

In the text, the terms "8782-nd site" and "28144-th site" refer to bases at the 8782-nd site and 28144-th site of sequences of the novel coronavirus genome with Genebank login No.: NC_045512.2.

In the present invention, the terms "sequences near 8782-nd site" and "sequences near 28144-th site" refer to sequences including the 8782 or 28144 site and including bases of 100-300 bp upstream and downstream thereof.

Furthermore, the sequences near the two sites are combined so that a mutation insertion sequence of the positive control can be obtained. For example, the insertion sequence (SEQ ID NO: 9) of C8782 and T28144 mutations of the 2019 novel coronavirus is:

TTAATTAAAGTTACACTTGTGTTCCTTTTTGTTGCTGCTATTTTCTATTTAATAACAC
CTGTTCATGTCATGTCTAAACATACTGACTTTTCAAGTGAAATCATAGGATACAAGGCT
ATTGATGGTGGTGTCACTCGTGACATAGCATCTACAGATACTTGTTTTGCTAACAAAC
ATGCTGATTTTGACACATGGTTTAGCCAGCGTGGTGGTAGTTATACTAATGACAAAGC
TTGCCCATTGATTGCTGCAGTCATAACAAGAGAAGTGGGTTTTGTCGTGCCTGGTTTG
CCTGGCACGATATTACGCACAACTAATGGTGACTTTTTGCATTTCTTACCTAGAGTTTT
TAGTGCAGTTGGTAACATCTGTTACACACCATCAAAACTTATAGAGTACACCAAGAAT
GTAGTTTACAGTCATGTACTCAACATCAACCATATGTAGTTGATGACCCGTGTCCTATT
CACTTCTATTCTAAATGGTATATTAGAGTAGGAGCTAGAAAATCAGCACCTTTAATTGA
ATTGTGCGTGGATGAGGCTGGTTCTAAATCACCCATTCAGTACATCGATATCGGTAATT
ATACAGTTTCCTGTTTACCTTTTACAATTAATTGCCAGGAACCTAAATTGGGTAGTCTT
GTAGTGCGTTGTTCGTTCTATGAAGACTTTTTAGAGTATCATGACGTTCGTGTTGTTTT
AGATTTCATCTAAACGAACAAACTAAAATGTCTGATAATGGACCCCAAAATCAGCGA
AATGCACCCCGCATTACGTTTGGTGGACCCTCAGATTCAA

The insertion sequence (SEQ ID NO: 10) of T8782 and C28144 mutations of the 2019 novel coronavirus is:

TTAATTAAAGTTACACTTGTGTTCCTTTTTGTTGCTGCTATTTTCTATTTAATAACAC
CTGTTCATGTCATGTCTAAACATACTGACTTTTCAAGTGAAATCATAGGATACAAGGCT
ATTGATGGTGGTGTCACTCGTGACATAGCATCTACAGATACTTGTTTTGCTAACAAAC
ATGCTGATTTTGACACATGGTTTAGTCAGCGTGGTGGTAGTTATACTAATGACAAAGC
TTGCCCATTGATTGCTGCAGTCATAACAAGAGAAGTGGGTTTTGTCGTGCCTGGTTTG
CCTGGCACGATATTACGCACAACTAATGGTGACTTTTTGCATTTCTTACCTAGAGTTTT
TAGTGCAGTTGGTAACATCTGTTACACACCATCAAAACTTATAGAGTACACCAAGAAT
GTAGTTTACAGTCATGTACTCAACATCAACCATATGTAGTTGATGACCCGTGTCCTATT

CACTTCTATTCTAAATGGTATATTAGAGTAGGAGCTAGAAAATCAGCACCTTTAAT
TGAATTGTGCGTGGATGAGGCTGGTTCTAAATCACCCATTCAGTACATCGATATCG
GTAATTATACAGTTTCCTGTTCACCTTTTACAATTAATTGCCAGGAACCTAAATTGG
GTAGTCTTGTAGTGCGTTGTTCGTTCTATGAAGACTTTTTAGAGTATCATGACGTT
CGTGTTGTTTTAGATTTCATCTAAACGAACAAACTAAAATGTCTGATAATGGACCC
CAAAATCAGCGAAATGCACCCCGCATTACGTTTGGTGGACCCTCAGATTCAA.

The positive control performs copy number quantification on two lentivirus samples by using a lentivirus digital PCR quantitative detection system. Two quantitative pseudoviruses are respectively prepared by diluting them to 1.0E+06 copy/mL with normal saline. The pseudoviruses take lentivirus as a vector and are inserted with two novel coronavirus genome sequences (8582-8982 and 27944-28344). Middle positions of the two sequences are respectively designed as bases corresponding to T8782 and C28144 mutations of the 2019 novel coronavirus and C8782 and T28144 mutations of the 2019 novel coronavirus.

In a fourth aspect, provided is a method for detecting mutations of 2019 novel coronavirus, including the following steps:
1) releasing nucleic acids from a to-be-tested sample;
2) using the composition of the present invention or the kit of the present invention to perform fluorescent quantitative PCR on the nucleic acids obtained in step 1); and
3) obtaining and analyzing a result.

In the present invention, a sample for detection can be a throat swab, sputum, bronchoalveolar lavage fluid, blood, etc., but is not limited thereto.

Furthermore, the reaction conditions of the fluorescent quantitative PCR are:
reverse transcription in which the temperature is 50°C, and the time is 25-35 minutes, 1 cycle; cDNApre-denaturation in which the temperature is 95°C, and the time is 1-10 minutes, 1 cycle; denaturation in which the temperature is 95°C, and the time is 10-20 seconds; and annealing in which the temperature is 60°C, and the time is 20-60 seconds, 40-50 cycles.

In a specific implementation, provided is a method for detecting mutations of 2019 novel coronavirus for non-diagnostic purposes, including the following steps:
1) releasing nucleic acids from a to-be-tested sample;
2) using the composition of the present invention or the kit of the present invention to perform fluorescent quantitative PCR on the nucleic acids obtained in step 1); and
3) obtaining and analyzing a result.

Furthermore, the reaction conditions of the fluorescent quantitative PCR are:
reverse transcription in which the temperature is 50°C, and the time is 25-35 minutes, 1 cycle; cDNA pre-denaturation in which the temperature is 95°C, and the time is 1-10 minutes, 1 cycle; denaturation in which the temperature is 95°C, and the time is 10-20 seconds; and annealing in which the temperature is 60°C, and the time is 20-40 seconds, 40-50 cycles.

In the text, the term "non-diagnostic purposes" refers to information that is not intended to obtain whether an individual is infected with the 2019 novel coronavirus and has pneumonia. For example, the method can detect the presence or absence of the 2019 novel coronavirus and virus mutation types in test cultures in experiments for research purposes.

### Brief Description of the Drawings

FIG. 1 shows amplification results of clinical nucleic acid samples detected by a first composition;
FIG. 2 shows amplification results of clinical nucleic acid samples detected by a second composition;
FIG. 3 shows results of C8782 and T28144 mutation templates (1.0E+08 copy/mL) of the 2019 novel coronavirus detected by the first composition;
FIG. 4 shows results of T8782 and C28144 mutations of the 2019 novel coronavirus and T8782 and C28144 mutation templates (1.0E+08 copy/mL) of the 2019 novel coronavirus detected by the first composition;
FIG. 5 shows results of T8782 and C28144 mutation templates (1.0E+08 copy/mL) of the 2019 novel coronavirus detected by the second composition;
FIG. 6 shows results of C8782 and T28144 mutation templates (1.0E+08 copy/mL) of the 2019 novel coronavirus detected by the second composition;
FIG. 7 shows results of C8782 and T28144 mutations of the 2019 novel coronavirus at gradient concentration and FAM channel detection detected by the first composition;
FIG. 8 shows results of C8782 and T28144 mutations of the2019 novel coronavirus at gradient concentration and HEX channel detection detected by the first composition;
FIG. 9 shows results of T8782 and C28144 mutation templates of the 2019 novel coronavirus at gradient concentration and FAM channel detection detected by the second composition;
FIG. 10 shows results of T8782 and C28144 mutation templates of the 2019 novel coronavirus at gradient concentration and HEX channel detection detected by the second composition;
FIG. 11 shows results of C8782 and T28144 mutation templates of the 2019 novel coronavirus (solid line) and T8782 and C28144 mutation templates of the 2019 novel coronavirus (dotted line) amplified by 8782 C-type primers in different designs, where FIG. 11A to FIG. 11F respectively relate to the results of the primers of the present invention and the primers 8782 C1-5 of the comparison examples;
FIG. 12 shows results of T8782 and C28144 mutation templates of the 2019 novel coronavirus (solid line) and C8782 and T28144 mutation templates of the 2019 novel coronavirus (dotted line) amplified by 8782 T-type primers in different designs, where FIG. 12A to FIG. 12F respectively relate to the results of the primers of the present invention and the primers 8782 T1-5 of the comparison examples;
FIG. 13 shows results of C8782 and T28144 mutation templates of the 2019 novel coronavirus (solid line) and T8782 and C28144 mutation templates of the 2019 novel coronavirus (dotted line) amplified by 28144 T-type primers in different designs, where FIG. 13A to FIG. 13F respectively relate to the results of the primers of the present invention and the primers 28144 T1-5 of the comparison examples; and
FIG. 14 shows results of T8782 and C28144 mutation templates of the 2019 novel coronavirus (solid line) and C8782 and T28144 mutation templates of the 2019 novel coronavirus (dotted line) amplified by 28144 C-type primers in different designs, where FIG. 14A to FIG. 14F respectively relate to the results of the primers of the present invention and the primers 28144 T1-5 of the comparison examples.

### Detailed Description

Hereinafter, the present invention is described in detail with reference to specific implementations and examples, and the advantages and various effects of the present invention may be more clearly presented therefrom. It should be understood by those skilled in the art that these specific implementations and examples are intended to illustrate the present invention, instead of limiting the present invention.

### Example 1. Primers and probes used in the present invention

Primers and probes used in the present invention are as shown below:
8782 C-type forward primer: 5'-GCTGATTTTGACACATGGTTCATC-3' (SEQ ID NO: 1);
28144 T-type forward primer: 5'-ATCGGTAATTATACAGTTTCCAGATT-3' (SEQ ID NO: 2);
8782 T-type forward primer: 5'-GCTGATTTTGACACATGGTATCGT-3' (SEQ ID NO: 3);
28144 C-type forward primer: 5'-ATCGGTAATTATACAGTTTCCTTTGC-3' (SEQ ID NO: 4);
8782 reverse primer: 5'-CCATTAGTTGTGCGTAATATCGT-3' (SEQ ID NO: 5).
8782 probe: 5'-CTTGCCCATTGATTGCTGCAGTCATAA-3' (SEQ ID NO: 6);
28144 reverse primer: 5'-CAACACGAACGTCATGATACTCTA-3' (SEQ ID NO: 7); and
28144 probe: 5'-ATTGCCAGGAACCTAAATTGGGTAGT-3' (SEQ ID NO: 8).

The fluorescent reporter group of the 8782 probe is HEX, and the fluorescent reporter group of the 28144 probe is FAM. The 3' end of the probe also has a quenching group of BHQ1 or BHQ2.

### Example 2. Method for detecting mutations of 2019 novel coronavirus

A sample for detection in the present invention is a throat swab, sputum, bronchoalveolar lavage fluid, or blood. The following operations are carried out:
(1) mixing a nucleic acid release agent S1014 of Sansure Biotech with a positive control at a ratio of 1:1, and lysing at room temperature for 10 minutes;
(2) according to the proportions of 26 µL of each reaction solution and 4 µL of mixed enzyme, taking a corresponding amount of reaction solution and mixed enzyme, mixing well, and dispensing 30 µL of each reaction into each reaction tube; adding 20 µL of the lysed to-be-tested sample to each reaction tube, then covering with a PCR tube cap, instantaneously centrifuging, and placing it in a real-time fluorescent PCR instrument; and
(3) performing reaction and detection under the following cycle conditions (a FAM channel and a HEX channel are selected for fluorescence acquisition):

| Step | Temperature | Time | Cycle number |
|---|---|---|---|
| Reverse transcription stage | 50°C | 30 minutes | 1 |
| cDNA pre-denaturation | 95°C | 1 minute | 1 |
| Denaturation | 95°C | 15 seconds | 45 |
| Annealing, extension and fluorescence acquisition | 60°C | 30 seconds | |

### Result analysis:

1) Target detection signals are FAM and HEX.
2) Baseline setting: the Baseline is generally set as 3-15 cycles, and can be adjusted according to actual situations. The adjusting principle is: selecting a region with relatively stable fluorescence signal before exponential amplification, the start avoiding signal fluctuation in an initial stage of fluorescence collection, and the end being 1-2 cycles less than the sample Ct with the earliest exponential amplification. Threshold setting: the setting principle is that a threshold line just exceeds the highest point of a normal negative control.
3) If there is no amplification signal in each channel of the two compositions, the sample concentration is lower than the detection lower limit.

If in two compositions, one and only one composition has amplification signals:
FAM and HEX of the first composition both have an amplification curve, and Ct < 40, representing C8782 and T28144 mutations of the 2019 novel coronavirus.
FAM and HEX of the second composition both have an amplification curve, and Ct < 40, representing T8782 and C28144 mutations of the 2019 novel coronavirus.

If an amplification signal exists in both of the two compositions, the difference between the Ct values of the corresponding channels in the two compositions are compared.

The difference of Ct values of the channels in the two compositions is greater than 10. If the Ct value of the first composition is relatively small, it is C8782 and T28144 mutations of the 2019 novel coronavirus. If the Ct value of the second composition is relatively small, it is T8782 and C28144 mutations of the 2019 novel coronavirus.

If the difference of Ct values of the channels in the two compositions is less than 10, the two types exist simultaneously.

For the first composition, when the FAM and HEX both have an amplification curve, and Ct < 35, it represents C8782 and T28144 mutations of the 2019 novel coronavirus.

For the second composition, when the FAM and HEX both have an amplification curve, and Ct < 35, it represents T8782 and C28144 mutations of the 2019 novel coronavirus.

For the first composition and the second composition, if the FAM and HEX have only one or no amplification curve, the type of the 2019 novel coronavirus cannot be determined. The determining result is as shown in Table 1 below.

The results of amplification in the two compositions are analyzed, where "+" refers to an amplification curve or Ct value less than 45, and "-" refers to no amplification curve or Ct value greater than 45. Ct₁ and Ct₂ are the Ct values of the corresponding channels in the first composition and the second composition, respectively; ΔCt is an absolute value of the difference between the two Ct values.

**Table 1**

| Channel | First composition | Second composition | Ct value | Result |
|---|---|---|---|---|
| FAM | - | - | | Low viral load |
| | + | - | | 28144 T-type |
| | - | + | | 28144 C-type |
| | + | + | ΔCt≥10, and Ct1 < Ct2 | 28144 T-type |
| | | | ΔCt≥10, and Ct1 > Ct2 | 28144 C-type |
| | | | ΔCt < 10 | 28144 C/T hybrid-type |
| HEX | - | - | | Low viral load |
| | + | - | | 8782 C-type |
| | - | + | | 8782 T-type |
| | + | + | ΔCt≥10, and Ct1 < Ct2 | 8782 C-type |
| | | | ΔCt≥10, and Ct1 > Ct2 | 8782 T-type |
| | | | ΔCt < 10 | 8782 C/T hybrid-type |

### Example 3. Composition of the present invention for detecting mutations of the 2019 novel coronavirus

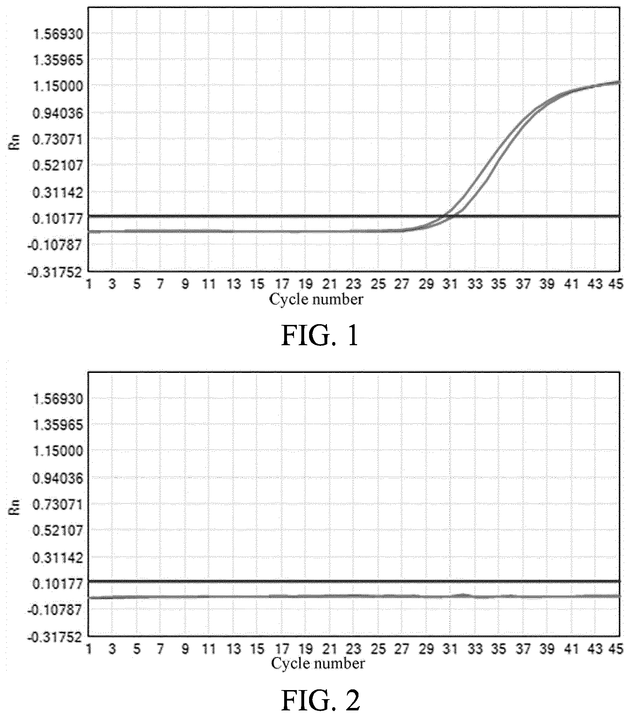

Detection is made using the composition in Example 1 of the present invention according to the method in Example 2. The sample is a nucleic acid extracted from clinically positive samples. The first composition and the second composition are respectively used for detection, and results thereof are as shown in FIG. 1 and FIG. 2. As can be seen from the figures that, the 8782-nd and 28144-th bases in this sample are C and T, respectively, which are C8782 and T28144 mutations of the 2019 novel coronavirus.

### Example 4. Specificity detection of the composition of the present invention

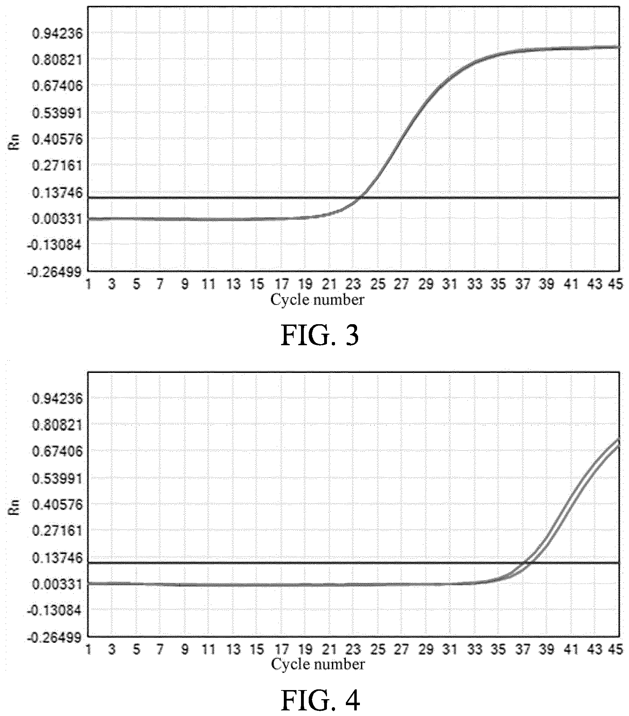

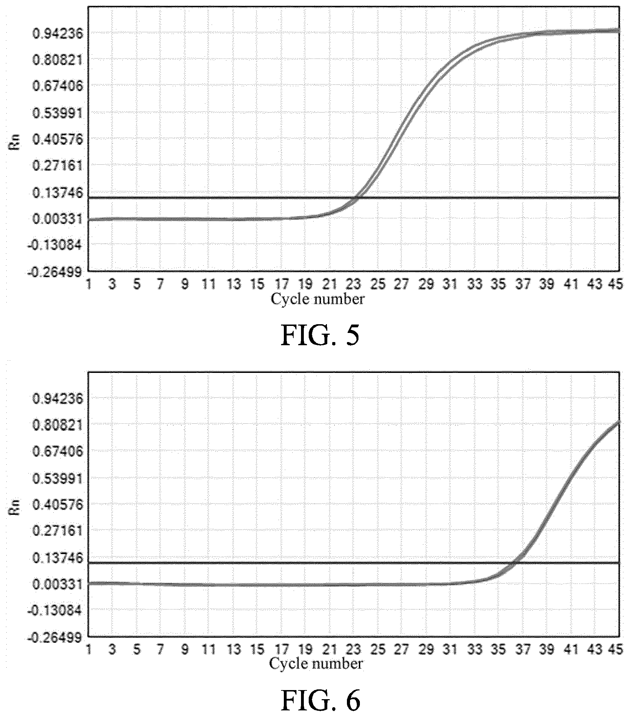

Detection is made using the composition in Example 1 of the present invention according to the method in Example 2. The samples are two pseudovirus samples with high concentration (the concentration is 1.0E+08 copy/mL). The first composition and the second composition are respectively used for detecting C8782 and T28144 mutations of the 2019 novel coronavirus and the pseudovirus templates of T8782 and C28144 mutations of the 2019 novel coronavirus. The results are as shown in FIG. 3 to FIG. 6. The Ct value of each detection result is as shown in Table 2 below.

**Table 2**

| T8782 and C28144 mutations of the 2019 novel coronavirus | Pseudovirus templates of C8782 and T28144 mutations of the 2019 novel coronavirus | Pseudovirus templates of T8782 and C28144 mutations of the 2019 novel coronavirus |
|---|---|---|
| First composition FAM | 23.09 | 37.06 |
| Second composition FAM | 36.13 | 23.48 |
| ΔCt-FAM | 13.04 | 13.58 |
| First composition HEX | 23.44 | 37.69 |
| Second composition HEX | 36.44 | 23.49 |
| ΔCt-HEX | 13.00 | 14.20 |

In view of the above, there are significant differences in amplification efficiency of different compositions against C8782 and T28144 mutation pseudoviruses of the 2019 novel coronavirus and T8782 and C28144 mutant pseudoviruses of the 2019 novel coronavirus. The minimum value of each ΔCt (13.00) is still much greater than the requirement (10) of ΔCt in the result determination method in Example 2.

Calculation is conducted based on the minimum value of ΔCt of 13.00 in each group, the amplification efficiency difference between the two compositions for the specific template and non-specific template is up to 8192 times (2^13), indicating excellent specificity.

### Example 5. Precision detection of the composition of the present invention

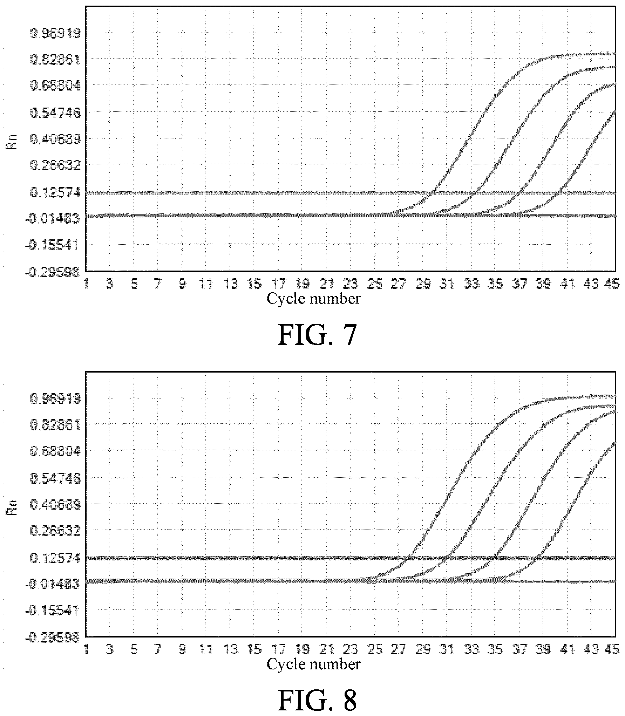

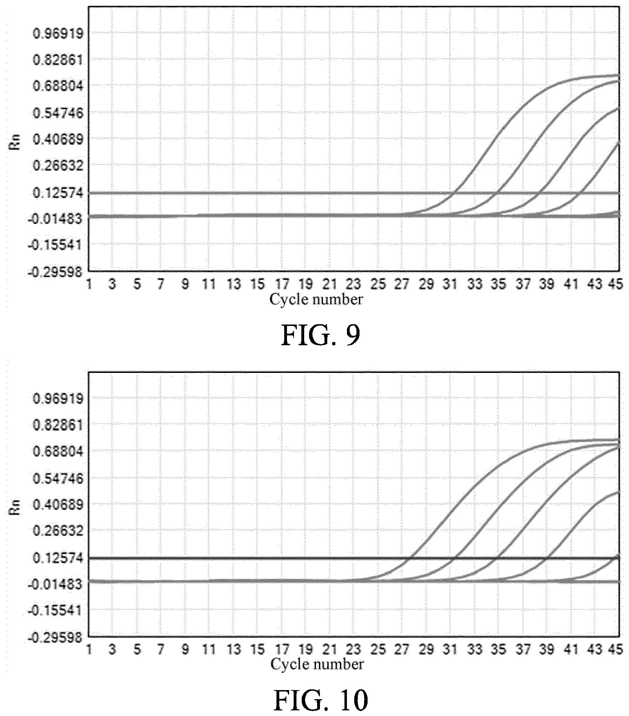

Detection is made using the composition in Example 1 of the present invention according to the method in Example 2. The samples are pseudovirus samples (C8782 and T28144 mutation pseudoviruses of the 2019 novel coronavirus and T8782 and C28144 mutation pseudoviruses of the 2019 novel coronavirus) which are diluted by normal saline. Each concentration gradient is separately 1.0E+06 copy/mL, 1.0E+05 copy/mL, 1.0E+04 copy/mL, 1.0E+03 copy/mL, 1.0E+02 copy/mL, and 1.0E+01 copy/mL. The first composition and the second composition are respectively used for detection of specific templates with different concentrations, and results thereof are as shown in FIG. 7 to FIG. 10.

The lowest detection concentration of the first composition is 1.0E+03 copy/mL (10 copy/reaction).

The lowest detection concentration of the second composition is 1.0E+03 copy/mL (10 copy/reaction).

### Comparison Example 1. Other probe and primer compositions of the present invention for detecting mutations of the 2019 novel coronavirus

In the creation process of the present invention, dozens of different specific recognition primers are designed for each site base. These primers all follow the design principle of ARMS primers, 1-2 mutations are added at the 3' end to selectively amplify specific targets. The sequences of some comparison primers are as follows:
8782 C-type forward comparison primers:
   8782 C-1: 5'-GCTGATTTTGACACATGGTTTAGC-3' (SEQ ID NO: 11);
   8782 C-2: 5'-GCTGATTTTGACACATGGTTTATC-3' (SEQ ID NO: 12);
   8782 C-3: 5'-GCTGATTTTGACACATGGTTTCGC-3' (SEQ ID NO: 13);
   8782 C-4: 5'-GCTGATTTTGACACATGGTTTCTC-3' (SEQ ID NO: 14); and
   8782 C-5: 5'-GCTGATTTTGACACATGGTCTCGT-3' (SEQ ID NO: 15);
8782 T-type forward comparison primers:
   8782 T-1: 5'-GCTGATTTTGACACATGGTTTAGT-3' (SEQ ID NO: 16);
   8782 T-2: 5'-GCTGATTTTGACACATGGTTTCGT-3' (SEQ ID NO: 17);
   8782 T-3: 5'-GCTGATTTTGACACATGGTTTATT-3' (SEQ ID NO: 18);
   8782 T-4: 5'-GCTGATTTTGACACATGGTTTCAT-3' (SEQ ID NO: 19); and
   8782 T-5: 5'-GCTGATTTTGACACATGGTTGATT-3' (SEQ ID NO: 20);
28144 T-type forward comparison primers:
   28144 T-1: 5'-ATCGGTAATTATACAGTTTCCTGTTT-3' (SEQ ID NO: 21);
   28144 T-2: 5'-ATCGGTAATTATACAGTTTCCTCTTT-3' (SEQ ID NO: 22);
   28144 T-3: 5'-ATCGGTAATTATACAGTTTCCTGGTT-3' (SEQ ID NO: 23);
   28144 T-4: 5'-ATCGGTAATTATACAGTTTCCTGGGT-3' (SEQ ID NO: 24); and
   28144 T-5: 5'-ATCGGTAATTATACAGTTTCCTCTCT-3' (SEQ ID NO: 25); and
28144 C-type forward comparison primers:
   28144 C-1: 5'-ATCGGTAATTATACAGTTTCCTGTTC-3' (SEQ ID NO: 26);
   28144 C-2: 5'-ATCGGTAATTATACAGTTTCCTCTTC-3' (SEQ ID NO: 27);
   28144 C-3: 5'-ATCGGTAATTATACAGTTTCCTGGTC-3' (SEQ ID NO: 28);
   28144 C-4: 5'-ATCGGTAATTATACAGTTTCCTGAGC-3' (SEQ ID NO: 29);
   28144 C-5: 5'-ATCGGTAATTATACAGTTTCCAGGTC-3' (SEQ ID NO: 30).

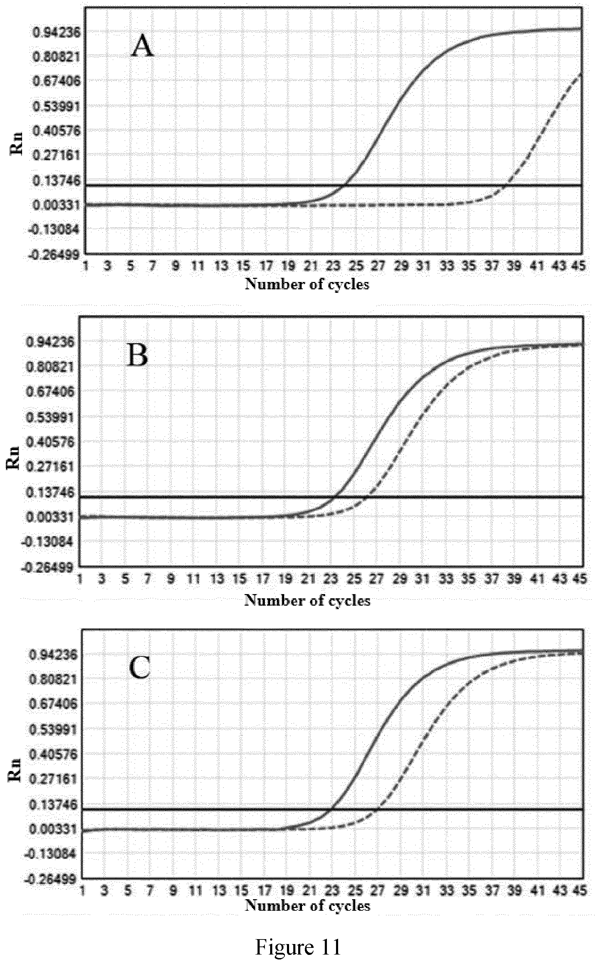

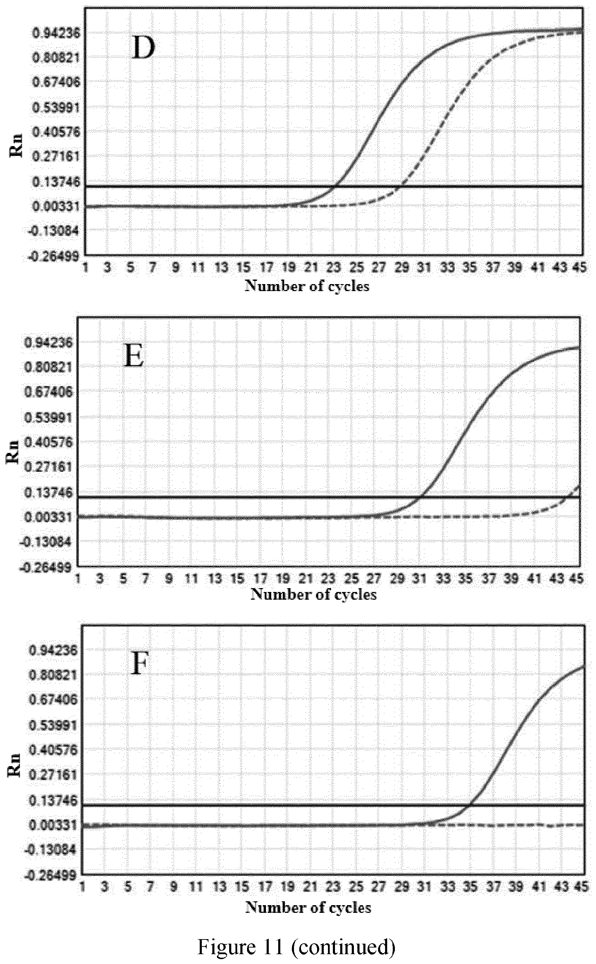

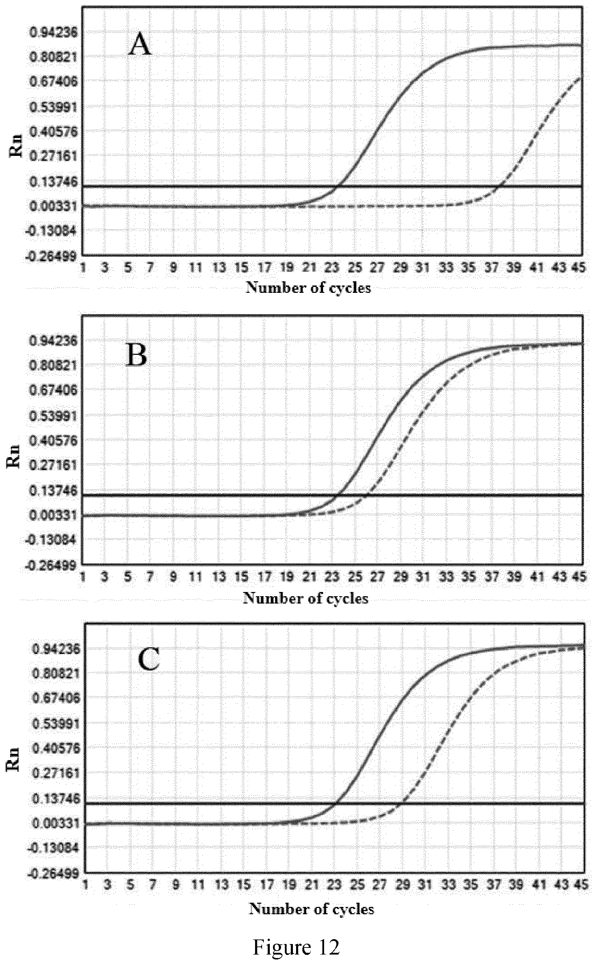

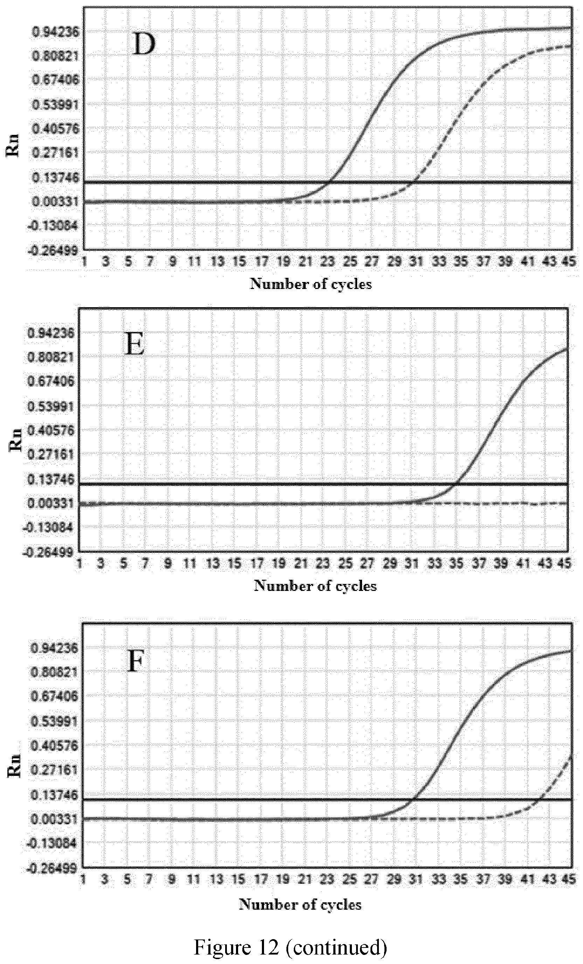

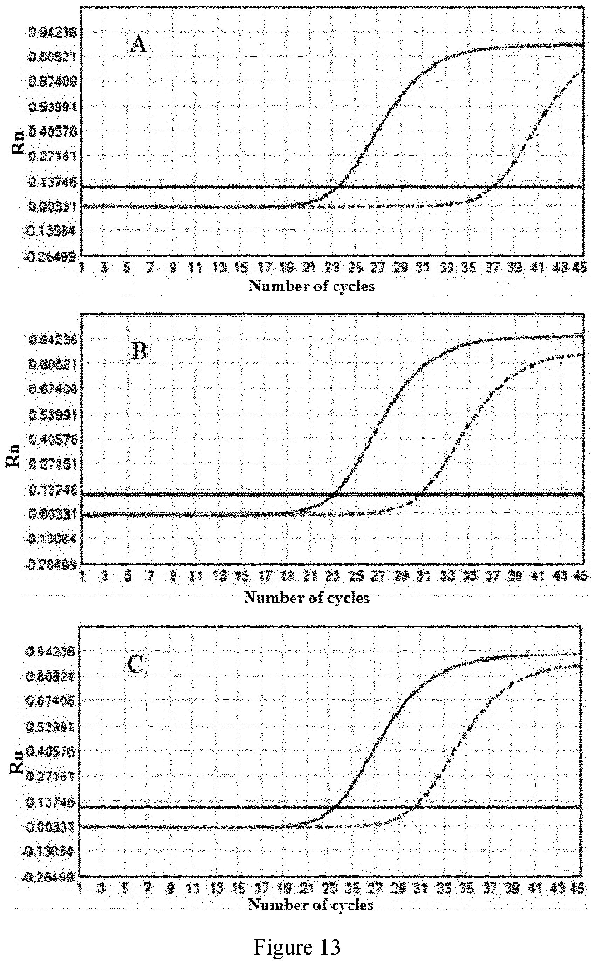

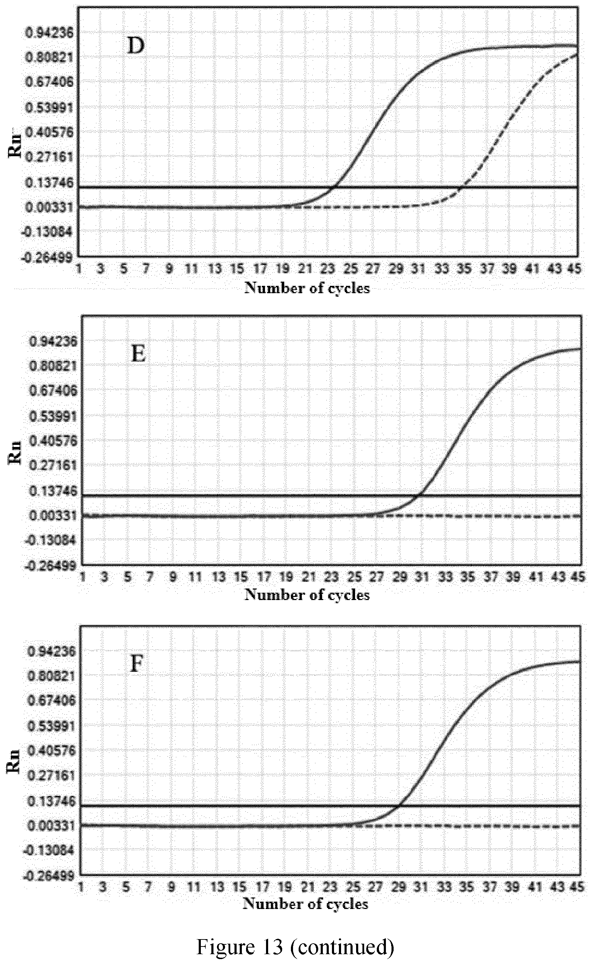

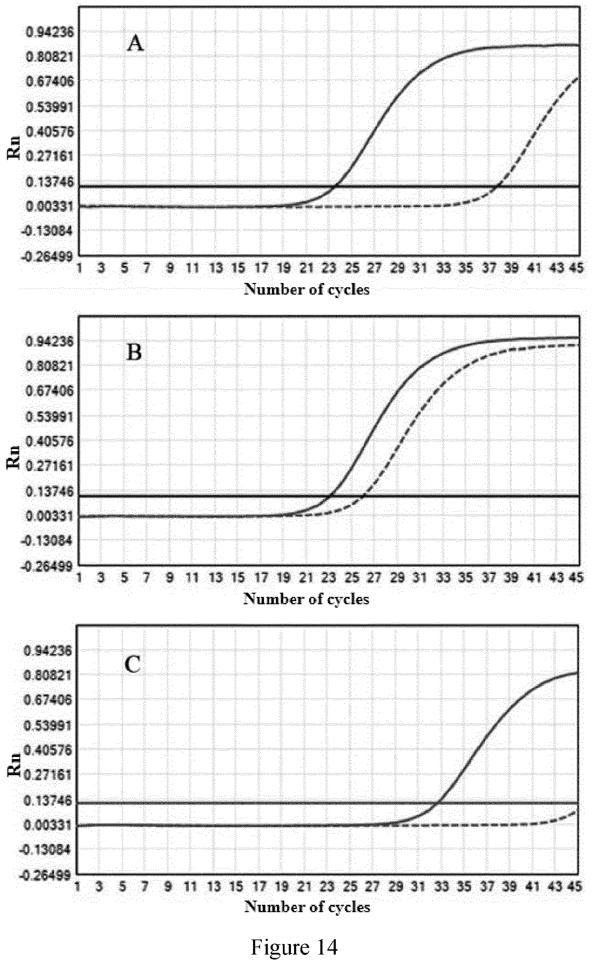

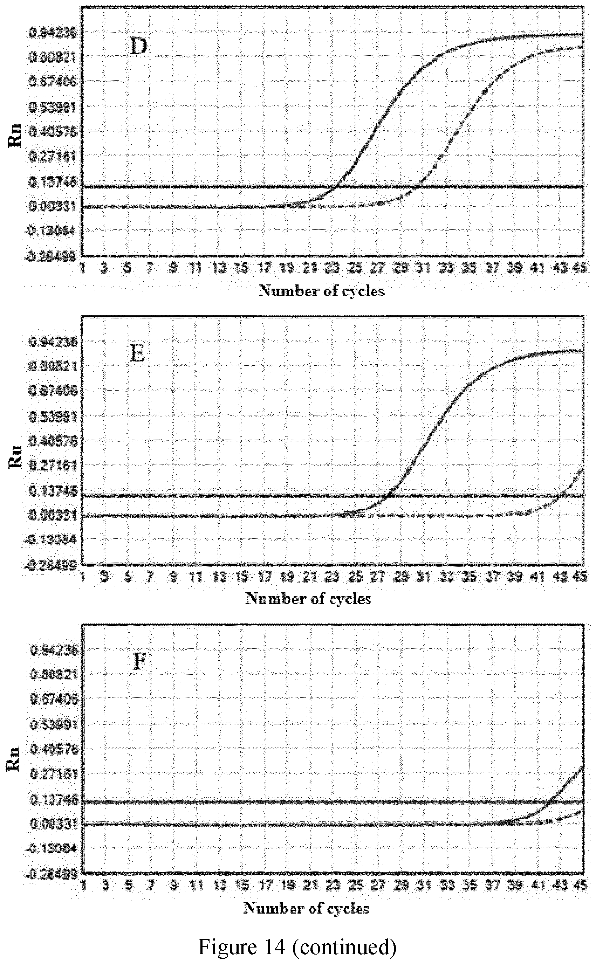

These primers are combined with other primers and probes in Example 1 to form compositions, and the method in Embodiment 2 is used for detection. The samples are two pseudovirus samples with the concentration of 1.0E+08 copy/mL. The test results are as shown in FIG. 11 to FIG. 14. In the result diagrams, the solid lines are the results of amplification of specific templates by each primer (for example, C8782 and T28144 mutation templates of the 2019 novel coronavirus detected by the 8782C primer, that is, the 8782 site in this template is C). The dotted lines are the results of amplification of non-specific templates by this primer (for example, T8782 and C28144 mutation templates of the 2019 novel coronavirus detected by the 8782C primer, that is, the 8782 site in this template is T).

Main indexes to evaluate the quality of the primers are the amplification efficiency and specificity of the primer. The smaller the Ct value of the primer detection amplification specific target is, the higher the amplification efficiency thereof is. When the Ct values of the specific template and the non-specific template at the same concentration are detected by the primer, the absolute value of the difference between the Ct values of the two templates is as large as possible, and the specificity thereof is better.

It can be seen from each result that the overall performances of the amplification efficiency and specificity of the comparative primers are inferior to those of the composition of the present invention.

## Claims

1. A composition for detecting mutations of 2019 novel coronavirus, comprising:
a first nucleic acid composition:
8782 C-type forward primer: 5'-GCTGATTTTGACACATGGTTCATC-3' (SEQ ID NO: 1); and
28144 T-type forward primer: 5'-ATCGGTAATTATACAGTTTCCAGATT-3' (SEQ ID NO: 2); and
a second nucleic acid composition:
8782 T-type forward primer: 5'-GCTGATTTTGACACATGGTATCGT-3' (SEQ ID NO: 3); and
28144 C-type forward primer: 5'-ATCGGTAATTATACAGTTTCCTTTGC-3' (SEQ ID NO: 4);
wherein each nucleic acid composition further comprises:
8782 reverse primer: 5'-CCATTAGTTGTGCGTAATATCGT-3' (SEQ ID NO: 5);
8782 probe: 5'-CTTGCCCATTGATTGCTGCAGTCATAA-3' (SEQ ID NO: 6);
28144 reverse primer: 5'-CAACACGAACGTCATGATACTCTA-3' (SEQ ID NO: 7); and
28144 probe: 5'-ATTGCCAGGAACCTAAATTGGGTAGT-3' (SEQ ID NO: 8).

2. The composition according to claim 1, wherein the 8782 probe and the 28144 probe carry fluorescent reporter groups that do not interfere with each other.

3. The composition according to claim 2, wherein the fluorescent reporter group is selected from a group consisting of FAM, HEX, ROX, VIC, CY5, 5-TAMRA, TET, CY3, and JOE.

4. The composition according to claim 1, wherein the fluorescent reporter group of the 8782 probe is HEX; and the fluorescent reporter group of the 28144 probe is FAM.

5. The composition according to claim 1, wherein the first nucleic acid composition and the second nucleic acid composition are in different packages.

6. The composition according to claim 1, further comprising an internal standard upstream primer, an internal standard downstream primer, and an internal standard probe for monitoring.

7. Use of the composition according to any one of claims 1 to 6 in preparation of a kit for detecting mutations of 2019 novel coronavirus.

8. A kit for detecting mutations of 2019 novel coronavirus, comprising the composition according to any one of claims 1 to 6.

9. The kit according to claim 8, comprising a positive control.

10. The kit according to claim 9, wherein the positive control comprises sequences near an 8782-nd site and sequences near a 28144-th site of a 2019 novel coronavirus genome, and wherein the 8782-nd site is C or T, and the 28144-th site is C or T.

## Patentansprüche

1. Zusammensetzung zur Erkennung von Mutationen des neuen 2019-Coronavirus, mit:
einer ersten Nukleinsäurezusammensetzung aufweisend:
einen 8782-C-Typ-Vorwärts-Primer: 5'-GCTGATTTTGACACATGGTTCATC-3' (SEQ-ID-Nr: 1); und
einen 28144-T-Typ-Vorwärts-Primer: 5'-ATCGGTAATTATACAGTTTCCAGATT-3' (SEQ-ID-Nr: 2); und
einer zweiten Nukleinsäurezusammensetzung aufweisend:
einen 8782-T-Typ-Vorwärts-Primer: 5'-GCTGATTTTGACACATGGTATCGT-3' (SEQ-ID-Nr: 3); und
einen 28144-C-Typ-Vorwärts-Primer: 5'-ATCGGTAATTATACAGTTTCCTTTGC-3' (SEQ-ID-Nr: 4);
wobei jede Nukleinsäurezusammensetzung ferner aufweist:
einen 8782-Reversierungsprimer: 5'-CCATTAGTTGTGCGTAATATCGT-3' (SEQ-ID-Nr: 5);
eine 8782-Sonde: 5'-CTTGCCCATTGATTGCTGCAGTCATAA-3' (SEQ-ID-Nr: 6);
einen 28144-Reversierungsprimer: 5'-CAACACGAACGTCATGATACTCTA-3' (SEQ-ID-Nr: 7); und
eine 28144-Sonde: 5'-ATTGCCAGGAACCTAAATTGGGTAGT-3' (SEQ-ID-Nr: 8).

2. Zusammensetzung nach Anspruch 1, wobei die 8782-Sonde und die 28144-Sonde fluoreszierende Meldegruppen tragen, die sich nicht gegenseitig stören.

3. Zusammensetzung nach Anspruch 2, wobei die fluoreszierende Meldegruppe ausgewählt ist aus einer Gruppe bestehend aus: FAM, HEX, ROX, VIC, CY5, 5-TAMRA, TET, CY3 und JOE.

4. Zusammensetzung nach Anspruch 1, wobei die fluoreszierende Meldegruppe der 8782-Sonde HEX ist; und die fluoreszierende Meldegruppe der 28144-Sonde FAM ist.

5. Zusammensetzung nach Anspruch 1, wobei die erste Nukleinsäurezusammensetzung und die zweite Nukleinsäurezusammensetzung in unterschiedlichen Packungen sind.

6. Zusammensetzung nach Anspruch 1, die ferner zur Überwachung einen internen standardmäßigen vorgeordneten Primer, einen internen standardmäßigen nachgeordneten Primer und eine interne standardmäßige Sonde aufweist.

7. Verwendung der Zusammensetzung nach einem der Ansprüche 1 bis 6 bei der Herstellung eines Kits zur Erkennung von Mutationen eines neuen 2019-Coronavirus.

8. Kit zur Erkennung von Mutationen eines neuen 2019-Coronavirus mit der Zusammensetzung nach einem der Ansprüche 1 bis 6.

9. Kit nach Anspruch 8, mit einer positiven Kontrolle.

10. Kit nach Anspruch 9, wobei die positive Kontrolle Sequenzen in der Nähe einer 8782-ten Stelle und Sequenzen in der Nähe einer 28144-ten Stelle eines neuen 2019-Coronavirusgenoms aufweist, und wobei die 8782-te Stelle C oder T ist und die 28144-te Stelle C oder T ist.

## Revendications

1. Composition pour la détection de mutations du nouveau coronavirus 2019, comprenant :
une première composition d'acide nucléique :
amorce sens 8782 de type C : 5'-GCTGATTTTGACACATGGTTCATC-3' (SEQ ID NO: 1) ; et
amorce sens 28144 de type T : 5'-ATCGGTAATTATACAGTTTCCAGATT-3' (SEQ ID NO: 2) ; et
une deuxième composition d'acide nucléique :
amorce sens 8782 de type T : 5'-GCTGATTTTGACACATGGTATCGT-3' (SEQ ID NO: 3) ; et
amorce sens 28144 de type C : 5'-ATCGGTAATTATACAGTTTCCTTTGC-3' (SEQ ID NO: 4) ;
dans laquelle chaque composition d'acide nucléique comprend en outre :
amorce antisens 8782 : 5'-CCATTAGTTGTGCGTAATATCGT-3' (SEQ ID NO: 5) ;
sonde 8782 : 5'-CTTGCCCATTGATTGCTGCAGTCATAA-3' (SEQ ID NO: 6) ;
amorce antisens 28144 : 5'-CAACACGAACGTCATGATACTCTA-3' (SEQ ID NO: 7) ; et
sonde 28144 : 5'-ATTGCCAGGAACCTAAATTGGGTAGT-3' (SEQ ID NO: 8).

2. Composition selon la revendication 1, dans laquelle la sonde 8782 et la sonde 28144 portent des groupes rapporteurs fluorescents qui n'interfèrent pas entre eux.

3. Composition selon la revendication 2, dans laquelle le groupe rapporteur fluorescent est sélectionné dans un groupe constitué par FAM, HEX, ROX, VIC, CY5, 5-TAMRA, TET, CY3, et JOE.

4. Composition selon la revendication 1, dans laquelle le groupe rapporteur fluorescent de la sonde 8782 est HEX ; et le groupe rapporteur fluorescent de la sonde 28144 est FAM.

5. Composition selon la revendication 1, dans laquelle la première composition d'acide nucléique et la deuxième composition d'acide nucléique sont dans des conditionnements différents.

6. Composition selon la revendication 1, comprenant en outre une amorce amont comme étalon interne, une amorce aval comme étalon interne, et une sonde comme étalon interne pour la surveillance.

7. Utilisation de la composition selon l'une quelconque des revendications 1 à 6 dans la préparation d'une trousse de détection de mutations du nouveau coronavirus 2019.

8. Trousse de détection de mutations du nouveau coronavirus 2019, comprenant la composition selon l'une quelconque des revendications 1 à 6.

9. Trousse selon la revendication 8, comprenant un témoin positif.

10. Trousse selon la revendication 9, dans laquelle le témoin positif comprend des séquences près d'un 8782^{ème} site et des séquences près d'un 28144^{ème} site d'un génome du nouveau coronavirus 2019, et dans laquelle le 8782^{ème} site est C ou T, et le 28144^{ème} site est C ou T.
